Europäisches Patentamt
European Patent Office
Office européen des brevets

(19)

(11) Veröffentlichungsnummer: **0 285 561**
**A2**

(12)

# EUROPÄISCHE PATENTANMELDUNG

(21) Anmeldenummer: **88810181.3**

(22) Anmeldetag: **21.03.88**

(51) Int. Cl.4: **C 07 D 493/12**
A 01 N 43/90, A 61 K 31/35
//(C07D493/12,313:00,311:00,
311:00,307:00)

(30) Priorität: **27.03.87 CH 1180/87**

(43) Veröffentlichungstag der Anmeldung:
**05.10.88 Patentblatt 88/40**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Gehret, Jean-Claude, Dr.**
**Im Aeschfeld 12**
**CH-4147 Aesch (CH)**

Die Bezeichnung der Erfindung wurde geändert (Richtlinien für die Prüfung im EPA, A-III, 7.3).

(54) **Parasitizide und insektizide Milbemycin-Derivate.**

(57) Neue Verbindungen der Formel

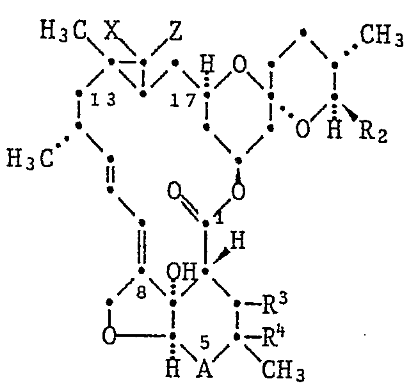

(I)

worin
A eine Gruppe

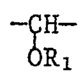 , 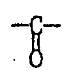 oder 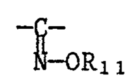

bedeutet, worin
$R_1$ für Wasserstoff oder eine OH-Schutzgruppe und $R_{11}$ für Wasserstoff, eine OH-Schutzgruppe, eine Alkyl-, Cycloalkyl oder Acylgruppe steht,
$R_2$ Methyl, Ethyl, Isopropyl, sek.Butyl oder die Gruppe $-C(CH_3)=CH-E$ bedeutet, worin E für Methyl, Ethyl oder Isopropyl steht,
$R_3$ und $R_4$ gemeinsam eine Bindung zwischen den beiden Kohlenstoffatomen, an die sie gebunden sind, oder gemeinsam die Gruppe $-C(X')(Z')-$, worin X' und Z' unabhängig voneinander für Wasserstoff oder Halogen stehen, und
X und Z unabhängig voneinander Wasserstoff oder Halogen bedeuten, die Herstellung der neuen Verbindungen und ihre Verwendung gegen Parasiten an Nutztieren und gegen Schadinsekten.

EP 0 285 561 A2

**Beschreibung**

Parasitizide und Insektizide

Die vorliegende Erfindung betrifft Milbemycin-Derivate der Formel I

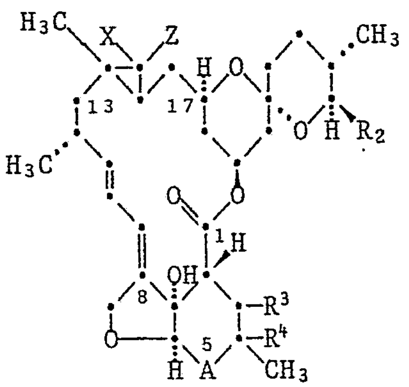

(I)

in welcher
A eine Gruppe

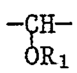 , 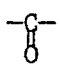 oder 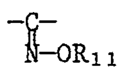

bedeutet, worin
$R_1$ für Wasserstoff oder eine OH-Schutzgruppe und $R_{11}$ für Wasserstoff, eine OH-Schutzgruppe, eine Alkyl-, Cycloalkyl- oder Acylgruppe steht,
$R_2$ Methyl, Ethyl, Isopropyl, sek.Butyl oder die Gruppe $-C(CH_3)=CH-E$ bedeutet, worin E für Methyl, Ethyl oder Isopropyl steht,
$R_3$ und $R_4$ gemeinsam eine Bindung zwischen den beiden Kohlenstoffatomen, an die sie gebunden sind, oder gemeinsam die Gruppe -C(X')(Z')-, worin X' und Z' unabhängig voneinander für Wasserstoff oder Halogen stehen, und
X und Z unabhängig voneinander Wasserstoff oder Halogen bedeuten.

Die Erfindung betrifft ferner die Herstellung der Verbindungen der Formel I und ihre Verwendung zur Bekämpfung von Nutztiere befallenden Parasiten oder Schadinsekten, sowie Mittel, die mindestens eine dieser Verbindungen als Wirkstoff enthalten.

Verbindungen, worin $R_2$ sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13α-Position) lassen sich jedoch gemäss US-PS 4,173,571 in Milbemycin-Homologe überführen.

Natürlich vorkommende Milbemycine ($R_1=H$; $R_2=CH_3$, $C_2H_5$ oder iso-$C_3H_7$) entsprechen nachstehender Formel M ($C_{14}$-$C_{15}$-Doppelbindung):

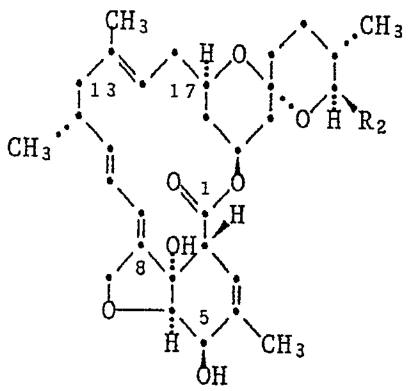

(M)

| | | |
|---|---|---|
| $R_2$ | $= CH_3$ | Milbemycin $A_3$ (US-PS 3,950,360) |
| $R_2$ | $= C_2H_5$ | Milbemycin $A_4$ (US-PS 3,950,360) |
| $R_2$ | $= isoC_3H_7$ | Milbemycin D (US-PS 4,346,171) |
| $R_2$ | $= sec.C_4H_9$ | 13-Deoxi-22,23-dihydro-C-076-Bla-aglycon (US-PS 4,173,571, GB 1,573,955 und DE-OS 2717040). |

Bei Avermectinen steht in der 13-Position ein α-L-Oleandrosyl-α-L-oleandrose-Rest, der über Sauerstoff in α-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen Substituenten $R_2$ = sek.$C_4H_9$ von den Milbemycinen. Durch Hydrolyse des Zucker-Restes der Avermectine gelangt man leicht zu den entsprechenden Avermectin-Aglykonen, die eine in Nachbarstellung zu einer C=C-Doppelbindung befindliche 13α-Hydroxy-Gruppe besitzen. Die Avermectin-Aglykone sind, wie vorstehend angegeben, in die Milbemycin-Homologen umwandelbar. Bei den Milbemycin-Derivaten der vorliegenden Anmeldung bilden das 22-C-Atom und das 23-C-Atom zusammen den Strukturteil $-CH_2-CH_2-$, wie er auch in der Formel M vorliegt.

Die Konstitution von natürlichen Antibiotika S541 ist aus der DE-OS-35 32 794 bekannt und sieht wie folgt aus:

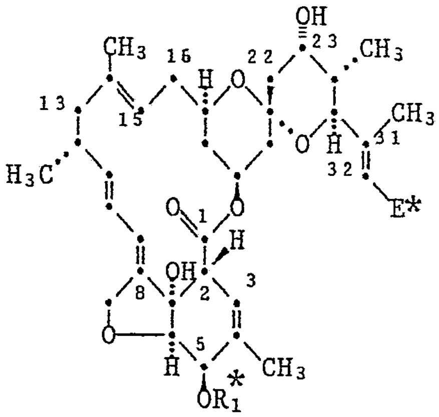

(Antibiotika S541)

| | | |
|---|---|---|
| Faktor A | $E^*=isoC_3H_7$ | $R_1^*=H$ |
| Faktor B | $E^*=CH_3$ | $R_1^*=CH_3$ |
| Faktor C | $E^*=CH_3$ | $R_1^*=H$ |
| Faktor D | $E^*=C_2H_5$ | $R_1^*=H$ |
| Faktor E | $E^*=C_2H_5$ | $R_1^*=CH_3$ |
| Faktor F | $E^*=isoC_3H_7$ | $R_1^*=CH_3$ |

Je nach Faktor werden im folgenden zur Vereinfachung der Benennung die Abkömmlinge von Antibiotikum S541 als Derivate von S541A, S541B, S541C, S541D, S541E oder S541F klassifiziert.

Verbindungen der Formel II, worin $R_2$ für die Gruppe $-\underset{CH_3}{C}=CH-E$ steht und E die für Formel II angegebene Bedeutung hat, welche als Ausgangsprodukte in dem erfindungsgemässen Verfahren verwendet werden können, lassen sich auf an sich bekannte Weise aus den natürlichen Antibiotika S541 herstellen.

Die in 23-Position befindliche Hydroxygruppe in den Antibiotika S541 lässt sich analog der in US-PS 4,328,335 beschriebenen Methode entfernen und die Antibiotika S541 lassen sich so in die entsprechenden 23-Deoxi-Derivate überführen. Dabei müssen diejenigen Verbindungen mit einer freien 5-OH-Gruppe ($R_1^*=H$) zunächst selektiv geschützt werden durch Reaktion mit einem der nachstehend angegebenen Silylierungsreagenzien $Y-Si(R_6)(R_7)(R_8)$ bzw. mit tert.-Butyldimethylsilyloxiacetylchlorid. Die Umsetzung dieser geschützten Verbindungen, in denen $R_1^*$ durch $Si(R_6)(R_7)(R_8)$ bzw. $C(=O)CH_2OSi(CH_3)_2t\text{-}C_4H_9$ ersetzt und das 23-C-Atom durch OH substituiert ist, mit p-Methylphenyl-chlorthionoformiat ergibt Derivate der Antibiotika S541, welche in Position 23 durch $p\text{-}CH_3\text{-}C_6H_4\text{-}O\text{-}C(=S)\text{-}O\text{-}$substituiert sind. Diese 23-O-(4-Methylphenoxi)-thiocarbonyl-derivate von Antibiotika S541 werden dann mit Tributylzinnhydrid in Toluol in Gegenwart von Azobisisobutyronitril bei 80-120°C zu den entsprechenden 23-Deoxi-Derivaten (Position 23 unsubstituiert) reduziert.

Verbindungen der Formel I, worin $R_2$ für Methyl, Ethyl, Isopropyl oder sek.Butyl steht, sind bevorzugt, insbesondere diejenigen, in denen A die Gruppen $-CH(OR_1)-$ und $-C(=N-OR_{11})-$und $R_1$ und $R_{11}$ Wasserstoff bedeuten.

Unter OH-Schutzgruppen für die Substituenten $R_1$ und/oder $R_{11}$ sollen hier und im folgenden die in der organischen Chemie üblichen Schutzfunktionen verstanden werden. Dabei handelt es sich insbesondere um Acyl- und Silylgruppen. Geeignete Acylgruppen sind beispielsweise die Reste $R_5\text{-}C(O)\text{-}$, wobei $R_5$ für $C_1\text{-}C_{10}$-Alkyl, $C_1\text{-}C_{10}$-Haloalkyl oder einen unsubstituierten oder durch mindestens einen Substituenten der Gruppe Halogen, $C_1\text{-}C_3$-Alkyl, $C_1\text{-}C_3$-Haloalkyl, $C_1\text{-}C_3$-Alkoxy, $C_1\text{-}C_3$-Haloalkoxy, Cyano und Nitro substituierten Vertreter der Gruppe Phenyl und Benzyl steht und vorzugsweise $C_1\text{-}C_6$-Alkyl, $C_1\text{-}C_6$-Haloalkyl oder unsubstituiertes oder durch Halogen, $C_1\text{-}C_3$-Alkyl, $CF_3$ oder Nitro substituiertes Phenyl bedeutet. Als geeignete Silylgruppen für $R_1$ kommt der Rest $-Si(R_6)(R_7)(R_8)$ in Frage, wobei $R_6$, $R_7$ und $R_8$ vorzugsweise unabhängig voneinander für $C_1\text{-}C_4$-Alkyl, Benzyl oder Phenyl stehen und beispielsweise zusammen mit dem Siliciumatom eine der Gruppen Trimethylsilyl, Diphenyl-tert.butylsilyl, bis(Isopropyl)-methylsilyl, Triphenylsilyl und insbesondere tert.Butyl-dimethylsilyl bildet. Die 5-OH-Gruppe kann auch verethert als Benzylether oder Methoxyethoxymethylether vorliegen oder gemäss der Europäischen Patentpublikation Nr. 185,623 an einen Kohlenhydratrest, im folgenden einfachheitshalber als Zuckerrest bezeichnet, gebunden sein.

In Verbindungen der Formel I, in denen $R_1$ für eine Silylgruppe, insbesondere tert.-Butyldimethylsilyl, oder

für eine Acylgruppe, beispielsweise eine Gruppe $R_5$-C(O)-, worin $R_5$ die vorstehend angegebenen Bedeutungen hat und insbesondere für Methyl steht, haben $R_2$, $R_3$, $R_4$, X und Z bevorzugt die für die Tabellen 1 und 2 geltenden Bedeutungen. In Verbindungen der Formel I, in denen $R_{11}$ für eine Silylgruppe, insbesondere tert.Butyldimethylsilyl, oder für eine Acylgruppe, beispielsweise eine Gruppe $R_5$-C(O)-, worin $R_5$ die vorstehend angegebenen Bedeutungen hat und insbesondere für Methyl steht, haben $R_2$, $R_3$, $R_4$, X und Z bevorzugt die für die Tabellen 5 und 6 geltenden Bedeutungen.

Verbindungen der Formel I, wobei $R_1$ und/oder $R_{11}$ eine Schutzgruppe darstellt, lasen sich durch einfache, z.B. hydrolytische Abspaltung der Schutzfunktion in die hochaktiven freien 5-Hydroxy-derivate ($R_1$ = H) oder 5-Hydroxyimino-derivate ($R_{11}$ = H) überführen und haben somit auch Zwischenprodukte-Charakter. Im übrigen wird der biologische Wert dieser Verbindungen durch die Schutzgruppe oder den Zuckerrest nicht gemindert.

Unter dem Begriff Alkyl als Substituent oder Teil eines Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl oder Decyl, sowie die Isomeren, wie beispielsweise Isopropyl, Isobutyl, tert.-Butyl oder Isopentyl. Wenn $R_{11}$ für eine Alkylgruppe steht, enthält diese vorzugsweise 1 bis 8, insbesondere 1 bis 4, Kohlenstoffatome.

Als Cycloalkyl-Gruppen kommen mono- bis tetracyclische Gruppen in Betracht, wie beispielsweise Cyclopropyl, Cyclobutyl, Cyclopentyl, Cyclohexyl, Decahydronaphthalin, Hydrindan, Bicycloheptan, Bicyclooctan, Norbornan, Bornan oder Adamantyl. Diese cycloaliphatischen Gruppen sind vorzugsweise unsubstituiert oder durch Methyl ein- oder mehrfach sustituiert. Stellt $R_{11}$ eine Cycloalkylgruppe dar, so enthält sie bevorzugt 3 bis 6 Kohlenstoffatome.

Die vorgenannten Acyl- und Silylgruppen dienen nicht nur als Schutzgruppen für im Substituenten A vorliegende Hydroxygruppen, sondern auch für alle anderen in den erfindungsgemässen Verbindungen oder Vorstufen dieser Verbindungen vorliegenden Hydroxygruppen.

Halogen in der Bedeutung von X, X', Z und Z' steht für Fluor, Chlor, Brom und Jod, bevorzugt für Fluor, Chlor und Brom. Vorzugsweise sind Halogenatome, die an dasselbe Kohlenstoffatom gebunden sind, miteinander identisch. Als Dihalogenmethylengruppen -C(X)(Z)- und -C(X')(Z')- sind Dichlormethylen und Dibrommethylen bevorzugt.

Folgende Untergruppen von Verbindungen der Formel I sind auf Grund ihrer ausgeprägten Wirkung gegen Schädlinge bevorzugt:

Gruppe Ia:

Verbindungen der Formel I, worin A eine der Gruppen -CH($OR_1$)-, -C(O)- oder -C(=N-OH)-, worin $R_1$ für Wasserstoff, eine Silylgruppe oder eine Monosaccharidgruppe steht, $R_2$ Methyl oder Ethyl, $R_3$ und $R_4$ gemeinsam eine Bindung zwischen den beiden Kohlenstoffatomen, an die sie gebunden sind, oder gemeinsam die Gruppe -C(X')(Z')-, worin X' und Z' unabhängig voneinander für Wasserstoff oder Halogen stehen, und X und Z unabhängig voneinander Wasserstoff oder Halogen bedeuten;

Gruppe Ib:

Verbindungen der Formel I, worin A eine der Gruppen -CH($OR_1$)-, -C(O)- oder -C(=N-OH)-, worin $R_1$ für Wasserstoff, Acetyl, tert.-Butyldimethylsilyl oder 2,3,4,6-Tetraacetylglukopyranosyl steht, $R_2$ Methyl oder Ethyl, $R_3$ und $R_4$ gemeinsam eine Bindung zwischen den beiden Kohlenstoffatomen, an die sie gebunden sind, oder gemeinsam die Gruppe -C($Cl_2$)-, X Wasserstoff, Chlor, Brom oder Fluor und Z Wasserstoff, Chlor, Brom oder Fluor bedeuten, wobei in Verbindungen, in denen X Chlor, Brom oder Fluor bedeutet, Z vorzugsweise mit X identisch ist.

Gruppe Ic:

Verbindungen der Formel I, worin A eine der Gruppen -CH($OR_1$)-, -C(O)- oder -C(=N-OH)-, worin $R_1$ für Wasserstoff, tert.-Butyldimethylsilyl oder 2,3,4,6-Tetraacetylglukopyranosyl steht, $R_2$ Methyl oder Ethyl, $R_3$ und $R_4$ gemeinsam eine Bindung zwischen den beiden Kohlenstoffatomen, an die sie gebunden sind, oder gemeinsam die Gruppe -C($Cl_2$)-, X Wasserstoff, Chlor oder Brom und Z Wasserstoff, Chlor oder Brom bedeuten, wobei in Verbindungen, in denen X Chlor oder Brom bedeutet, Z vorzugsweise mit X identisch ist.

Gruppe Id:

Verbindungen der Formel I, worin A die Gruppe -CH(OH)-, oder -C(=N-OH)-, $R_2$ Methyl oder Ethyl, $R_3$ und $R_4$ gemeinsam eine Bindung zwischen den beiden Kohlenstoffatomen, an die sie gebunden sind, X Wasserstoff, Chlor, Brom oder Fluor und Z Wasserstoff, Chlor, Brom oder Fluor bedeuten.

Gruppe Ie:

Verbindungen der Formel I, worin A die Gruppe -CH(OH)-oder -C(=N-OH)-, $R_2$ Methyl oder Ethyl, $R_3$ und $R_4$ gemeinsam eine Bindung zwischen den beiden Kohlenstoffatomen, an die sie gebunden sind, und X und Z miteinander identisch sind und Wasserstoff, Chlor oder Brom oder X Wasserstoff und Z Chlor oder Brom bedeuten.

Gruppe If:

Verbindung der Formel I, in denen A die Gruppe -CH(OH)-, $R_2$ Methyl oder Ethyl, $R_3$ und $R_4$ gemeinsam eine Bindung zwischen den beiden Kohlenstoffatomen, an die sie gebunden sind oder gemeinsam die Gruppe $-CCl_2-$, X Wasserstoff, Chlor oder Brom und Z Wasserstoff, Chlor oder Brom bedeuten.

Im Rahmen der vorliegenden Anmeldung wird das Strukturelement

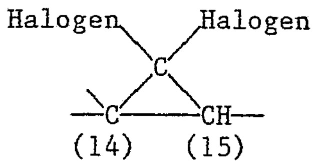

durch die Bezeichnung "14,15-Dihalogenmethylen-14,15-dihydro" definiert;
das Strukturelement

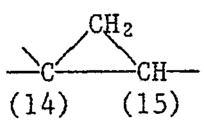

durch die Bezeichnung "14,15-Methylen-14,15-dihydro" definiert; das Strukturelement

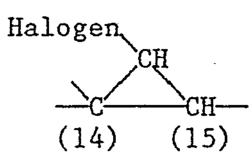

durch die Bezeichnung "14,15-Monohalogenmethylen-14,15-dihydro" definiert;
das Strukturelement

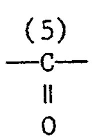

durch die Bezeichnung "5-Oxo" definiert;
das Strukturelement

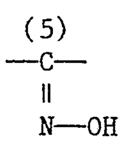

durch die Bezeichnung "5-Hydroxyimino" definiert.
Die Bezeichnungen für die gegebenenfalls halogenierte $C_3$-$C_4$-Methylengruppe sind analog denjenigen für $C_{14}$-$C_{15}$ angegeben.

Bevorzugte Einzelverbindungen sind:
5-O-(tert.-Butyldimethylsilyl)-14,15-dichlormethylen-14,15-dihydromilbemycin $A_4$,
5-O-(tert.-Butyldimethylsilyl)-3,4-dichlormethylen-14,15-dichlormethylen-3,4,14,15-tetrahydromilbemycin $A_4$,
3,4-Dichlormethylen-14,15-dichlormethylen-3,4,14,15-tetrahydromilbemycin $A_4$,
5-O-(tert.-Butyldimethylsilyl)-14,15-dibrommethylen-14,15-dihydromilbemycin $A_4$,
5-O-(2,3,4,6-Tetraacetylglukopyranosyl)-14,15-dichlormethylen-14,15-dihydromilbemycin $A_4$,
5-Oxo-14,15-dichlormethylen-14,15-dihydromilbemycin $A_4$,
5-Hydroxyimino-14,15-dichlormethylen-14,15-dihydromilbemycin $A_4$,
14,15-Dichlormethylen-14,15-dihydromilbemycin $A_3$,
5-O-(tert.-Butyldimethylsilyl)-14,15-monobrommethylen-14,15-dihydromilbemycin $A_4$,
5-O-(tert.-Butyldimethylsilyl)-14,15-methylen-14,15-dihydromilbemycin $A_4$,
14,15-Methylen-14,15-dihydromilbemycin $A_4$ und insbesondere
14,15-Dibrommethylen-14,15-dihydromilbemycin $A_4$,
14,15-Dichlormethylen-14,15-dihydromilbemycin $A_4$,
14,15-Monobrommethylen-14,15-dihydromilbemycin $A_4$ und
14,15-Monochlormethylen-14,15-dihydromilbemycin $A_4$.

Die Herstellung von Verbindungen der Formel I kann analog solchen Methoden erfolgen, wie sie für die Addition von Carbenen an olefinisch ungesättigte Strukturen in der Carbene betreffenden Literatur beschrieben sind, beispielsweise in Liebigs Ann. Chem., 744, 42-50 (1971) und in Organic Chemistry, Vol. 1, "Carbene Chemistry", W. Kirmse, Academic Press, New York, London, 1971, Part II: "Structure and Reactivity of Carbenes and Carbenoids", insbesondere Kapitel 8, unter Einschluss der in der vorgenannten Literatur zitierten weiterführenden Literatur.

Zur Herstellung von Verbindungen der Formel I setzt man eine Verbindung der Formel II

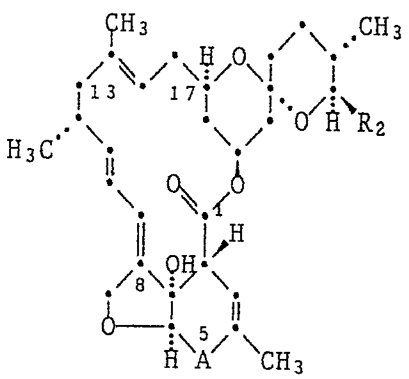

(II)

in welcher A und $R_2$ die für die Formel I angegebenen Bedeutungen haben, mit einem in situ gebildeten, in einem reaktionsinerten Lösungsmittel gelösten Carben der Formel IIIa

C(X)(Z) (IIIa)

worin X und Z die für Formel I angegebenen Bedeutungen haben, um, und setzt gewünschtenfalls eine so erhaltene Verbindung der Formel I, in welcher $R_3$ und $R_4$ gemeinsam eine Bindung zwischen den Kohlenstoffatomen, an die sie gebunden sind, darstellen, mit einem in situ gebildeten, in einem reaktionsinerten Lösungsmittel gelösten Carben der Formel IIIb

C(X′)(Z′) (IIIb)

worin X′ und Z′ die für Formel I angegebenen Bedeutungen haben, um. Als reaktionsinerte Lösungsmittel kommen beispielsweise 1,2-Dimethoxyethan, bis(2-Methoxyethyl)-ether, Acetonitril, Dichlormethan, Chloroform, Dichlorethan und Alkane, wie Pentan oder Hexan, in Betracht. Temperatur und Reaktionszeit ergeben sich weitgehend aus den Bedingungen der gewählten Carbenherstellung. Im allgemeinen liegen die Temperaturen in einem Bereich von -70° C bis + 180° C, vorzugsweise 0° C bis 40° C, und die Reaktionszeiten variieren in einem Bereich von etwa 10 Minuten bis zu 2 Tagen. Während bei niedrigen Temperaturen vorzugsweise Verbindungen der Formel I entstehen, in denen $R_3$ und $R_4$ gemeinsam eine Bindung zwischen den beiden Kohlenstoffatomen, an die sie gebunden sind, darstellen (Monoaddukt), überwiegt bei höheren Temperaturen der Anteil and denjenigen Verbindungen, in denen $R_3$ und $R_4$ gemeinsam die Gruppe -C(X′)(Z′)-bilden (Diaddukt).

Die Erzeugung von Carbenen der Formeln IIIa und IIIb kann auf konventionelle Weise erfolgen, wie

beispielsweise
- aus einem Quecksilbersalz, wie beispielsweise Phenyl(trifluoromethyl)-quecksilber bei ca. 80°C;
- aus Natriumdifluorchloracetat bei 100 bis 140°C;
- aus Trichloressigsäure-ethylester und einer Base, wie beispielsweise Natriummethoxyd bei ca. 0-20°C;
- aus Chloroform oder Bromoform und einer Base (beispielsweise 30-50 % wässriger Natronlauge, Kalium-tert.-butylat oder Butyllithium) mit oder ohne Phasentransferkatalysator (wie beispielsweise Tetraalkylammoniumchloride oder -bromide), bei Temperaturen von ca. -40 bis +60°C, wobei Chloroform oder Bromoform gleichzeitig als Lösungsmittel dienen (liefert Dihalogencarbene);
- aus Methylenchlorid oder Methylenbromid und einer Base (beispielsweise 30-50 % wässriger Natronlauge, Kalium-tert.-butylat oder Butyllithium) mit oder ohne Phasentransferkatalysatoren (wie beispielsweise Tetraalkylammoniumchloride oder -bromide), bei Temperaturen von ca. -40°C bis +60°C, wobei Methylenchlorid oder Methylenbromid als Lösungsmittel dienen können (liefert Monohalogencarbene);
- aus Chloroform, Bromoform, Methylenchlorid oder Methylenbromid mit festem Natrium- oder Kaliumhydroxid und Beschallung mit Ultraschall.

Durch Reduktion lassen sich in Verbindungen der Formel I Mono- und Dihalogenmethylengruppen in unsubstituierte Methylengruppen sowie Dihalogenmethylengruppen in Monohalogenmethylengruppen überführen. Die Reduktion kann analog bekannten Methoden, beispielsweise mit Tributylzinnhydrid oder Zink und Säure, erfolgen.

Verbindung der Formel I lassen sich beispielsweise erhalten, indem man entweder ein Milbemycin der Formel M oder das 23-Deoxi-Derivat von S541A, S541C oder S541D in eine Verbindung der Formel II, in welcher A eine andere Bedeutung als -CH(OH)- hat, umwandelt und anschliessend mit einem Carben umsetzt, oder indem man eine Verbindung der Formel M oder das 23-Deoxi-Derivat von S541A, S541C oder S541D zunächst mit einem Carben umsetzt und die so erhaltene Verbindung in eine Verbindung der Formel I, in welcher A eine andere Bedeutung als -CH(OH)- hat, überführt.

Ferner können in Verbindungen der Formeln I und II am 5-C-Atom vorliegende Substituenten abgespalten und gewünschtenfalls durch andere Substituenten ersetzt werden, soweit diese den erfindungsgemässen Definitionen entsprechen. Die Abspaltungen und Einführungen definitionsgemässer Substituenten können nach an sich bekannten Methoden erfolgen. Zur Einführung von Acyl-, Silyl- und Saccharidgruppen geht man zweckmässigerweise von Verbindungen der Formeln I und II, in denen A für -CH(OH)- oder -C(=N-OH)- steht, oder von Verbindungen der Formel M oder von den 23-Deoxi-Derivaten von S541A, S541C oder S541D aus. Die Herstellung von Verbindungen der Formeln I oder II, in denen A für -C(=N-$OR_{11}$)-steht, kann beispielsweise erfolgen, indem man Verbindungen der Formeln I oder II, in denen A für -C(O)- steht, mit Hydroxylamin oder einem seiner Salze umsetzt und gewünschtenfalls anschliessend den Substituenten $R_{11}$, wobei $R_{11}$ die für Formel I angegebenen Bedeutungen mit Ausnahme von Wasserstoff hat, einführt, oder indem man die Umsetzung mit einer Verbindung der Formel $NH_2$-$OR_{11}$, worin $R_{11}$ die für Formel I angegebenen Bedeutungen mit Ausnahme von Wasserstoff hat, oder einem Salz davon vornimmt. Als Salze kommen beispielsweise solche der vorgenannten Aminoverbindungen mit Schwefelsäure, Salpetersäure und vor allem Salzsäure in Betracht. Die Reaktion wird zweckmässigerweise in einem geeigneten Lösungsmittel ausgeführt, z.B. einem Niederalkanol, wie Methanol, Ethanol, Propanol; einer etherartigen Verbindung, wie Tetrahydrofuran oder Dioxan; einer aliphatischen Carbonsäure, wie Essigsäure oder Propionsäure; Wasser oder in Gemischen dieser Lösungsmittel untereinander oder mit anderen üblichen reaktionsinerten Lösungsmitteln. Die Reaktionstemperaturen können in weiten Bereichen variieren. Man arbeitet zweckmässigersweise etwa im Bereich von +10° bis +100°C. Wird Hydroxylamin in Form eines seiner Salze, z.B. als Hydrochlorid eingesetzt, so ist es vorteilhaft, wenn man zum Abfangen der Säure eine der für solche Zwecke üblichen Basen zusetzt und gegebenenfalls in Gegenwart eines Wasserbinders, z.B. eines Molekularsiebes, arbeitet. Als geeignete Basen kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali-und Erdalkalimetallen (CaO, BaO, NaOH, KOH, NaH, $Ca(OH)_2$, $KHCO_3$, $NaHCO_3$, $Ca(HCO_3)_2$, $K_2CO_3$, $Na_2CO_3$), sowie Alkaliacetate wie $CH_3COONa$ oder $CH_3COOK$. Darüberhinaus eignen sich auch Alkalialkoholate wie $C_2H_5ONa$, n-$C_3H_7ONa$ usw. Bevorzugt wird Triethylamin.

Verbindungen der Formeln I und II, in denen A für -C(O)- steht, lassen sich beispielsweise gewinnen, indem man Verbindungen der Formeln I oder II, in denen A für -CH(OH)- steht, mit einem zur Oxidation geeigneten Reagenz behandelt. Geeignete Oxydationsmittel sind beispielsweise aktiviertes Mangandioxyd, Oxalylchlorid/Dimethylsulfoxyd/Triethylamin oder Chromtrioxyd/Pyridin. Ein geeignetes Verfahren stellt auch die Oppenauer-Oxydation dar, bei welcher Verbindungen der Formeln I und II, in denen A für -CH(OH)- steht, mit einem Keton, vorzugsweise Cyclohexanon oder Aceton, in Gegenwart eines Aluminiumalkoholats, vorzugsweise des Aluminiumisopropylats oder Aluminium-tert.-butylats, umsetzt.

Die Oxydation wird zweckmässigerweise in einem inerten Lösungsmittel durchgeführt. Als geeignete Lösungsmittel kommen Alkane, wie beispielsweise Hexan, Heptan oder Oktan, aromatische Kohlenwasserstoffe, wie beispielsweise Benzol, Toluol oder Xylole, oder bevorzugt chlorierte Kohlenwasserstoffe, insbesondere Methylenchlorid, in Frage. Die Oxydation wird zweckmässigerweise bei Temperaturen von -80°C bis +60°C, vorzugsweise -60°C bis +30°C. durchgeführt.

Aus Verbindungen der Formeln I und II, in denen A für die Gruppe -C(O)- steht, lassen sich durch Reduktion auf an sich bekannte Weise wieder diejenigen Verbindungen gewinnen, in denen A für die Gruppe -CHOH-

steht. Die Reduktion kann beispielsweise durch katalytische Hydrierung mit Platin- oder Raney-Nickel-Katalysator oder gemäss der Meerwein-Ponndorf-Verley-Reduktion mit Aluminiumisopropylat in Isopropanol erfolgen.

Die Einführung eines Saccharids in Verbindungen der Formel M, in 23-Deoxi-Derivate von S541A, S541C oder S541D oder in Verbindungen der Formeln I und II, in denen A für -CH(OH)- steht, lässt sich durch Umsetzung dieser Verbindungen mit dem entsprechenden Saccharid durchführen, wobei man zweckmässigerweise analog Methoden, wie sie in der Zuckerchemie für Verknüpfungsreaktionen allgemein bekannt sind, vorgeht, wie beispielsweise nach der Koenigs-Knorr-Methode, nach dem Ag-Triflat-Prozess, nach dem sogenannten Ortho ester-Verfahren, nach der Phenylthio-Synthese oder nach der 2-Pyridylthio-Methode (gemäss der Europäischen Offenlegungsschrift Nr. 185,623).

Zur Herstellung von Verbindungen der Formel II, in denen $R_1$ für eine Acylgruppe steht, wird die 5-OH-Gruppe eines Milbemycins der Formel M oder eines 23-Deoxi-Derivats von S541A, S541C oder S541D acyliert. Die Einführung der Acylgruppe erfolgt üblicherweise mit den entsprechenden Acylhalogeniden oder Acylanhydriden, wobei der Begriff Acylhalogenid für Acylchlorid oder Acylbromid steht, und wird vorzugsweise zur Einführung der eingangs definierten $R_5C(O)$-Gruppe benutzt.

Zur Herstellung von Verbindungen der Formel II, in denen $R_1$ für eine Silylgruppe steht, wird die 5-OH-Gruppe eines Milbemycins der Formel M oder eines 23-Deoxi-Derivats von S541A, S541C oder S541D silyliert. Zur Silylierung verwendet man zweckmässigerweise ein Silan der Formel $Y\text{-}Si(R_6)(R_7)(R_8)$, worin $R_6$, $R_7$ und $R_8$ die vorgängig genannten Bedeutungen haben und Y eine Silylabgangsgruppe bedeutet. Zu den Silylabgangsgruppen Y zählen beispielsweise Brom, Chlor, Cyan, Azido, Acetamid, Trifluoracetoxy und Trifluormethansulfonyloxy. Diese Aufzählung stellt keine Limitierung dar, der Fachmann kennt weitere typische Silylabgangsgruppen.

5-O-Acylierungen und 5-O-Silylierungen werden in wasserfreiem Milieu, vorzugsweise in inerten Lösungsmitteln und besonders bevorzugt in aprotischen Lösungsmitteln, durchgeführt. Die Reaktion läuft vorteilhaft im Temperaturbereich von 0° bis +80°C, bevorzugt bei +10° bis +40°C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage.

Die Entfernung dieser Silyl- und Acylreste $R_1$ oder $R_{11}$ in der 5-Position geschieht durch selektive milde Hydrolyse ($\rightarrow$ $R_1$ oder $R_{11}$ = H) beispielsweise mit verdünnten Säuren wie verdünnter HCl, HF, Arylsulfonsäure in alkoholischer oder wässriger Lösung oder nach einer anderen dem Fachmann geläufigen Methode. Acylreste werden vorzugweise basisch (z.B. in alkoholischer Ammoniaklösung) abgespalten.

Das erfindungsgemässe Verfahren zur Herstellung von Verbindungen der Formel I, worin A, $R_2$, $R_3$, $R_4$, X und Z die für Formel I angegebenen Bedeutungen haben, besteht darin, dass man eine Verbindung der Formel II

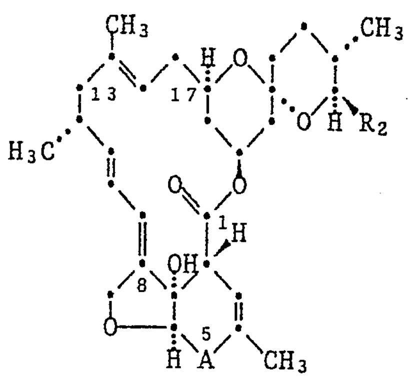

(II)

in welcher A und $R_2$ die für Formel I angegebenen Bedeutungen haben, mit einem in situ gebildeten, in einem reaktionsinerten Lösungsmittel gelösten Carben der Formel IIIa

C(X)(Z) (IIIa)

worin X und Z die für Formel I angegebenen Bedeutungen haben, umsetzt und in erhaltenen Verbindungen gewünschtenfalls i) eine für -C(X)(Z)- stehende Gruppe -C(Halogen)(Halogen)- zur Gruppe -CH(Halogen)- oder $-CH_2-$ reduziert oder ii) eine für -C(X)(Z)-stehende Gruppe -CH(Halogen)- zur Gruppe $-CH_2-$ reduziert oder iii) die für A stehende Gruppe in eine andere der unter A definierten Gruppen überführt, oder erhaltene Verbindungen, in denen $R_3$ und $R_4$ gemeinsam eine Bindung zwischen den beiden Kohlenstoffatomen, an die sie gebunden sind, darstellen, mit einem in situ gebildeten, in einem reaktionsinerten Lösungsmittel gelösten

Carben der Formel IIIb

C(X')(Z') (IIIb)

worin X' und Z' die für Formel I angegebenen Bedeutungen haben, umsetzt und in erhaltenen Verbindungen gewünschtenfalls iv) eine für -C(X)(Z)- oder -C(X')(Z')- stehende Gruppe -C(Halogen)(Halogen)-zur Gruppe -CH(Halogen)- oder -$CH_2$- reduziert oder v) eine für -C(X)(Z)-oder -C(X')(Z')- stehende Gruppe -CH(Halogen)-zur Gruppe -$CH_2$-reduziert oder vi) die für A stehende Gruppe in eine andere der unter A definierten Gruppen überführt.

Das beschriebene Verfahren zur Herstellung der Verbindungen der Formel I ist ein Bestandteil vorliegender Erfindung.

Die Verbindungen der Formel I eignen sich ausgezeichnet zur Bekämpfung von Schädlingen an Tieren und Pflanzen, darunter insbesondere von tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbare gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksame gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Coccidae, Diaspididae und Eriophydidae (z.B. die Rostmilbe auf Citrusfrüchten): der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rhizoglyphus und andere.

Ferner sind die Verbindungen gegen alle Entwicklungsstadien von Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Asc ris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Gattungen Nematodirus, Cooperia und Oesophagostomum greifen den Intestinaltrakt des Wirtstiers an, während andere der Gattungen Haemonchus und Ostertagia im Magen und solche der Gattung Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Weiter sind die Verbindungen der Formel I zur Bekämpfung von humanpathogenen Parasiten geeignet, unter denen als typische, im Verdauungstrakt vorkommende Vertreter solche der Gattungen Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Gattungen Wuchereria, Brugia, Onchocerca und Loa aus der Familie der Filariidae, die im Blut, im Gewebe und verschiedenen Organen vorkommen, ferner gegen Dracunculus und Parasiten der Gattungen Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht dosiert. Ueber geschlossenen Kultur-Anbauflächen werden sie in Mengen von 10 g bis 100 g pro Hektar angewendet. Sie kommen ferner in Pferchen, Gehegen, Stallungen oder sonstigen Räumen zur Anwendung.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome in Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro PropylenglykolEinheit 1 bis 5 Aethylenglykoleinheiten.

Als Beispiele nichtionischer Tenside seinen Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben: "1986 International McCutcheon's Emulsifiers and Detergents" The Manufacturing Confectioner Publishing Co., Glen Rock, New Jersey, USA.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als Wirkstoff mindestens eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

Beispiel H-1: Herstellung von 5-O-(tert.-Butyldimethylsilyl)-14,15-dichlormethylen-14,15-dihydromilbemycin $A_4$ und 5-O-(tert.-Butyldimethylsilyl)-3,4-dichlormethylen-14,15-dichlormethylen-3,4,14,15-tetrahydromilbemycin $A_4$

Zu einer Lösung von 1300 mg 5-O-(tert.-Butyldimethylsilyl)-milbemycin $A_4$ und 15 mg Tetrabutylammoniumchlorid in 60 ml Chloroform werden unter ständigem Rühren bei 0 bis 5°C 15 ml einer 50 %igen wässrigen Natriumhydroxydlösung gegeben. Nach 30 Minuten wird das Reaktionsgemisch mit 200 ml Essigsäureethylester verdünnt und mit Wasser/Kochsalzlösung neutral gewaschen. Nach Trocknung der Lösung über Natriumsulfat und Eindampfen wird den Rückstand über Silikagel (Hexan: Ether = 5:1) gereinigt. Nach Gefriertrocknung des Produkts erhält man 1090 mg 5-O-(tert.-Butyldimethylsilyl)-14,15-dichlor methylen-14,15-dihydromilbemycin $A_4$, Smp. 102-105°C, und 80 mg 5-O-(tert.-Butyldimethylsilyl)-3,4-dichlormethylen-14,15-dichlormethylen-3,4,14,15-tetrahydromilbemycin $A_4$; Smp. ca. 200°C.

Aus 5-O-(tert.-Butyldimethylsilyl)-3,4-dichlormethylen-14,15-dichlormethylen-3,4,14,15-tetrahydromilbemycin $A_4$ erhält man durch Abspaltung der Silylgruppe 3,4-Dichlormethylen-14,15-dichlormethylen-3,4,14,15-tetrahydromilbemycin $A_4$; Smp. 153-157°C.

Beispiel H-2: Herstellung von 14,15-Dichlormethylen-14,15-dihydromilbemycin $A_4$

120 mg 5-O-(tert.-Butyldimethylsilyl)-14,15-dichlormethylen-14,15-dihydromilbemycin $A_4$ in 5 ml einer 1 %igen methanolischen Lösung von p-Toluolsulfonsäure werden eine Stunde bei Raumtemperatur gerührt und anschliessend mit 5 %iger wässriger Natriumhydrogencarbonatlösung behandelt. Nach dreimaligem Ausschütteln mit je 20 ml Diethylether, Trocknen über Natriumsulfat, Einengen der organischen Phase und Chromatographie des Rohproduktes an 20 g Kieselgel (Laufmittel: Diethylether) erhält man 92 mg 14,15-Dichlormethylen14,15-dihydromilbemycin $A_4$; Smp. 127-131°C.

Aus 14,15-Dichlormethylen-14,15-dihydromilbemycin $A_4$ erhält man durch Umsetzung mit 2,3,4,6-Tetraacetylglukopyranose 5-O-(2,3,4,6-Tetraacetylglukopyranosyl)-14,15-dichlormethylen-14,15-dihydromilbemycin $A_4$; Smp. 133-138°C (Verbindung Nr. 7.1).

Beispiel H-3: Herstellung von 14,15-Dichlormethylen-14,15-dihydromilbemycin $A_3$

Zu einer Lösung von 400 mg (0.75 mmol) Milbemycin $A_3$ und 5 mg Tetrabutylammoniumchlorid in 50 ml Chloroform werden unter ständigem Rühren bei 0 bis 3°C 7 ml einer 50 %igen wässrigen Natriumhydroxydlösung zugegeben. Nach 15 Minuten wird das Reaktionsgemisch mit 100 ml Diethylether verdünnt und mit Wasser/Kochsalzlösung bis zum Erhalt einer neutralen Reaktion gewaschen. Die Lösung wird über Natriumsulfat getrocknet und eingedampft. Der so erhaltene Rückstand wird über eine Silika-Kolonne (Hexan: Ether = 4:1) gereinigt. Nach Gefriertrocknen des erhaltenen Produkts erhält man 212 mg 14,15-Dichlormethylen-14,15-dihydromilbemycin $A_3$; Smp. 130-135°C.

Beispiel H-4: Herstellung von 14,15-Dibrommethylen-14,15-dihydromilbemycin $A_4$

100 mg Milbemycin $A_4$ und 100 mg Magnesiumspäne in 5 ml Diethylether werden bei Raumtemperatur mit 0.5 ml Bromoform versetzt. Die Reaktionsmischung, in welcher nach einigen Minuten eine exotherme Reaktion einisetzt, wird durch Kühlen mit einem Eisbad bei 30 bis 35°C gehalten, bis nach etwa einer Stunde die Reaktion beendet ist. Das Reaktionsgemisch wird filtriert und eingeengt. Die säulenchromatographische Reinigung des Rohproduktes (Kieselgel; Diethylether/Hexan = 10:1) ergibt 78 mg 14,15-Dibrommethylen-14,15-dihydromilbemycin $A_4$, welches sich bei 141-144°C zersetzt.

Aus 14,15-Dibrommethylen-14,15-dihydromilbemycin $A_4$ erhält man durch Einführung der Gruppe $-Si(CH_3)_2$-tert.-$C_4H_9$ 5-O-(tert.-Butyldimethylsilyl)-14,15-dibrommethylen-14,15-dihydromilbemycin $A_4$; Smp. 137-141°C.

Beispiel H-5: Herstellung von 5-O-(tert.-Butyldimethylsilyl)-14,15-dichlormethylen-14,15-dihydromilbemycin $A_4$

Zu 100 mg 5-O-(tert.-Butyldimethysilyl)-milbemycin $A_4$ in 4 ml trockenem Chloroform wird unter Argon bei -60°C 1 ml n-Butyl-Lithium zugetropft. Das Reaktionsgemisch wird langsam auf 20°C erwärmt, 4 Stunden intensiv gerührt, dann filtriert und eingeengt. Das so erhaltene Rohprodukt wird säulenchromatographisch gereinigt (Kieselgel; Diethylether/Petrolether = 1:5). Man erhält 90 mg 5-O-(tert.-Butyldimethylsilyl)-14,15-dichlormethylen-14,15-dihydromilbemycin $A_4$; Smp. 103-105°C.

Beispiel H-6: Herstellung von 5-O-(tert.-Butyldimethylsilyl)-14,15-monobrommethylen-14,15-dihydromilbemycin $A_4$ und 5-O-(tert.-Butyldimethylsilyl)-14,15-methylen-14,15-dihydromilbemycin $A_4$

Zu einer Lösung von 200 mg (0,24 mmol) 5-O-(tert.-Butyldimethylsilyl)-14,15-dibrommethylen-14,15-dihydromilbemycin $A_4$ in 10 ml Eisessig werden bei 15°C langsam 2,0 g Zinkpulver zugegeben. Nach 4 Stunden wird das Lösungsmittel am Vakuum abgedampft und der Rückstand filtriert und über einer Kieselgelsäule gereinigt (Cyclohexan:Essigsäureethylester = 12:1). Nach Gefriertrocknung erhält man 70 mg 5-O-(tert.-Butyldimethylsilyl)-14,15-monobrommethylen-14,15-dihydromilbemycin $A_4$ als amorphes Pulver, Smp. 180-185°C, und 100 mg 5-O-(tert.-Butyldimethylsilyl)-14,15-methylen-14,15-dihydromilbemycin $A_4$ in amorpher Form, welches bei ca. 75°C schmilzt.

Beispiel H-7: Herstellung von 14,15-Monobrommethylen-14,15-dihydromilbemycin $A_4$

70 mg 5-O-(tert.-Butyldimethylsilyl)-14,15-monobrommethylen-14,15-dihydromilbemycin $A_4$ werden bei Raumtemperatur mit 2 ml 1 %iger methanolischer p-Toluolsulfonsäurelösung versetzt. Nach Aufarbeitung gemäss vorstehenden Beispielen erhält man 14,15-Monobrommethylen-14,15-dihydromilbemycin $A_4$ als amorphes Pulver, welches bei 85-88°C schmilzt.

Beispiel H-8: Herstellung von 14,15-Methylen-14,15-dihydromilbemycin $A_4$

100 mg 5-O-(tert.-Butyldimethylsilyl)-14,15-methylen-14,15 dihydromilbemycin $A_4$ werden bei Raumtemperatur mit 2 ml 1 %iger methanolischer p-Toluolsulfonsäurelösung versetzt. Nach Aufarbeitung gemäss vorstehenden Beispielen erhält man 14,15-Methylen-14,15-dihydromilbemycin $A_4$ als amorphes Pulver, welches bei 80-83°C schmilzt.

Aus 14,15-Dichlormethylen-14,15-dihydromilbemycin $A_4$ lässt sich durch Oxydation zu 5-Oxo-14,15-dichlormethylen-14,15-dihydromilbemycin $A_4$ und Umsetzung dieser 5-Oxo-Verbindung mit Hydroxylamin 5-Hydroxyimino-14,15-dichlor-14,15-dihydro-milbemycin $A_4$ herstellen; Smp. 171-173°C.

Analog zu den beschriebenen Arbeitsweisen werden auch die nachfolgend zusammen mit Verbindungen der vorgängigen Beispiele genannten Verbindungen der Formel I hergestellt, wobei die Aufstellung jedoch keinen limitierenden Charakter hat.

Tabelle 1:

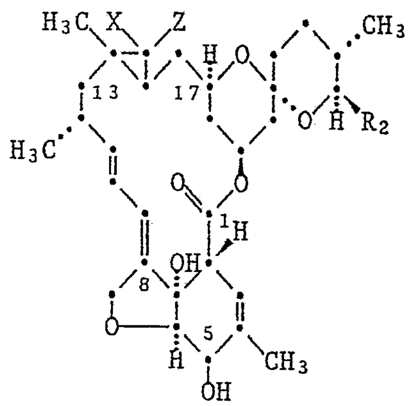

| Verb. Nr. | $R_2$ | X | Z |
|---|---|---|---|
| 1.1 | $CH_3$ | H | Br |
| 1.2 | $C_2H_5$ | H | Br |
| 1.3 | $C_3H_7$-iso | H | Br |
| 1.4 | $C_4H_9$-sek | H | Br |
| 1.5 | $CH_3$ | H | Cl |
| 1.6 | $C_2H_5$ | H | Cl |
| 1.7 | $C_3H_7$-iso | H | Cl |
| 1.8 | $C_4H_9$-sek | H | Cl |
| 1.9 | $CH_3$ | H | H |
| 1.10 | $C_2H_5$ | H | H |
| 1.11 | $C_3H_7$-iso | H | H |
| 1.12 | $C_4H_9$-sek | H | H |
| 1.13 | $CH_3$ | Cl | Cl |
| 1.14 | $C_2H_5$ | Cl | Cl |
| 1.15 | $C_3H_7$-iso | Cl | Cl |
| 1.16 | $C_4H_9$-sek | Cl | Cl |
| 1.17 | $CH_3$ | Br | Br |
| 1.18 | $C_2H_5$ | Br | Br |
| 1.19 | $C_3H_7$-iso | Br | Br |
| 1.20 | $C_4H_9$-sek | Br | Br |
| 1.21 | $CH_3$ | H | F |
| 1.22 | $C_2H_5$ | J | J |
| 1.23 | $C_3H_7$-iso | Cl | Br |
| 1.24 | $C_4H_9$-sek | H | J |
| 1.25 | $CH_3$ | Cl | Br |
| 1.26 | $C_2H_5$ | F | F |
| 1.27 | $C_3H_7$-iso | H | F |
| 1.28 | $C_4H_9$-sek | Cl | J |
| 1.29 | $CH_3$ | F | F |
| 1.30 | $C_2H_5$ | Cl | Br |
| 1.31 | $C_3H_7$-iso | Br | F |
| 1.32 | $C_4H_9$-sek | H | F |
| 1.33 | $CH_3$ | Br | J |
| 1.34 | $C_2H_5$ | H | F |
| 1.35 | $C_3H_7$-iso | F | F |
| 1.36 | $C_4H_9$-sek | Cl | Br |
| 1.37 | $CH_3$ | F | J |
| 1.38 | $C_2H_5$ | H | J |

Tabelle 1 (Fortsetzung)

| Verb. Nr. | $R_2$ | X | Z |
|---|---|---|---|
| 1.39 | $C_3H_7$-iso | Cl | J |
| 1.40 | $C_4H_9$-sek | Br | F |
| 1.41 | $CH_3$ | H | J |
| 1.42 | $C_2H_5$ | Cl | F |
| 1.43 | $C_3H_7$-iso | Br | J |
| 1.44 | $C_4H_9$-sek | J | J |
| 1.45 | $CH_3$ | Br | F |
| 1.46 | $C_2H_5$ | Cl | J |
| 1.47 | $C_3H_7$-iso | H | J |
| 1.48 | $C_4H_9$-sek | F | F |
| 1.49 | $CH_3$ | Cl | J |
| 1.50 | $C_2H_5$ | Br | F |
| 1.51 | $C_3H_7$-iso | Cl | F |
| 1.52 | $C_4H_9$-sek | Br | J |
| 1.53 | $CH_3$ | J | J |
| 1.54 | $C_2H_5$ | Br | F |
| 1.55 | $C_3H_7$-iso | F | J |
| 1.56 | $C_4H_9$-sek | F | J |
| 1.57 | $CH_3$ | Cl | F |
| 1.58 | $C_2H_5$ | F | J |
| 1.59 | $C_3H_7$-iso | J | J |
| 1.60 | $C_4H_9$-sek | Cl | F |

sowie die Verbindungen der Tabelle 1, in denen das Wasserstoffatom der in 5-Position befindlichen Hydroxygruppe durch die Silylgruppe $-Si(CH_3)_2$-tert.-$C_4H_9$ ersetzt ist (= Verbindungen S-1.1 bis S-1.60) und die Verbindungen der Tabelle 1, in denen das Wasserstoffatom der in 5-Position befindlichen Hydroxygruppen durch die Acylgruppe $-COCH_3$ ersetzt ist (= Verbindungen A-1.1 bis A-1.60).

Tabelle 2:

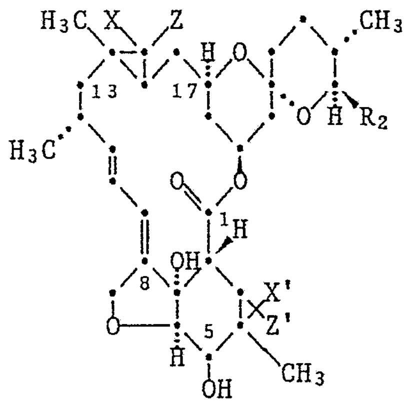

| Verb. Nr. | $R_2$ | X | Z | X' | Z' |
|---|---|---|---|---|---|
| 2.1 | $CH_3$ | H | Br | H | Br |
| 2.2 | $C_2H_5$ | H | Br | H | Br |
| 2.3 | $C_3H_7$-iso | H | Br | H | Cl |
| 2.4 | $C_4H_9$-sek | H | Br | H | Cl |
| 2.5 | $CH_3$ | H | Cl | H | H |
| 2.6 | $C_2H_5$ | H | Cl | H | Cl |
| 2.7 | $C_3H_7$-iso | H | Cl | H | H |
| 2.8 | $C_4H_9$-sek | H | Cl | H | Br |
| 2.9 | $CH_3$ | H | H | H | H |
| 2.10 | $C_2H_5$ | H | H | Cl | Cl |
| 2.11 | $C_3H_7$-iso | H | H | Br | Br |
| 2.12 | $C_4H_9$-sek | H | H | H | H |
| 2.13 | $CH_3$ | Cl | Cl | Cl | Cl |
| 2.14 | $C_2H_5$ | Cl | Cl | Cl | Cl |
| 2.15 | $C_3H_7$-iso | Cl | Cl | Br | Br |
| 2.16 | $C_4H_9$-sek | Cl | Cl | J | J |
| 2.17 | $CH_3$ | Br | Br | H | H |
| 2.18 | $C_2H_5$ | Br | Br | Br | Br |
| 2.19 | $C_3H_7$-iso | Br | Br | Br | Br |
| 2.20 | $C_4H_9$-sek | Br | Br | H | H |
| 2.21 | $CH_3$ | H | F | H | F |
| 2.22 | $C_2H_5$ | J | J | J | J |
| 2.23 | $C_3H_7$-iso | Cl | Br | Cl | Cl |
| 2.24 | $CH_3$ | Cl | Br | Cl | Br |
| 2.25 | $C_2H_5$ | F | F | F | F |
| 2.26 | $CH_3$ | F | F | Cl | Cl |
| 2.27 | $C_2H_5$ | Cl | Br | Cl | Br |
| 2.28 | $C_4H_9$-sek | H | F | H | H |
| 2.29 | $C_2H_5$ | H | F | H | F |
| 2.30 | $C_3H_7$-iso | F | F | F | F |
| 2.31 | $C_4H_9$-sek | Cl | Br | Cl | Cl |
| 2.32 | $C_2H_5$ | H | J | H | Cl |

Tabelle 2 (Fortsetzung)

| Verb. Nr. | $R_2$ | X | Z | X' | Z' |
|---|---|---|---|---|---|
| 2.33 | $CH_3$ | H | J | H | J |
| 2.34 | $C_2H_5$ | Cl | F | Cl | F |
| 2.35 | $CH_3$ | J | J | Cl | Cl |
| 2.36 | $CH_3$ | Cl | F | Cl | Br |
| 2.37 | $C_2H_5$ | F | J | H | H |

sowie die Verbindungen der Tabelle 2, in denen das Wasserstoffatom der in 5-Position befindlichen Hydroxygruppe durch die Silylgruppe $-Si(CH_3)_2$-tert.-$C_4H_9$ ersetzt ist (= Verbindungen S-2.1 bis S-2.37) und die Verbindungen der Tabelle 2, in denen das Wasserstoffatom der in 5-Position befindlichen Hydroxygruppe durch die Acylgruppe $-COCH_3$ ersetzt ist (=Verbindungen A-2.1 bis A-2.37).

Tabelle 3:

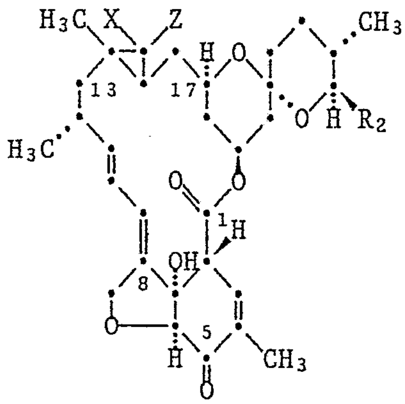

| Verb. Nr. | $R_2$ | X | Z |
|---|---|---|---|
| 3.1 | $CH_3$ | H | H |
| 3.2 | $C_2H_5$ | H | H |
| 3.3 | $C_3H_7$-iso | H | H |
| 3.4 | $C_4H_9$-sek | H | H |
| 3.5 | $CH_3$ | Br | Br |
| 3.6 | $C_2H_5$ | Br | Br |
| 3.7 | $C_3H_7$-iso | Br | Br |
| 3.8 | $C_4H_9$-sek | Br | Br |
| 3.9 | $CH_3$ | Cl | Cl |
| 3.10 | $C_2H_5$ | Cl | Cl |
| 3.11 | $C_3H_7$-iso | Cl | Cl |
| 3.12 | $C_4H_9$-sek | Cl | Cl |
| 3.13 | $CH_3$ | H | Br |
| 3.14 | $C_2H_5$ | H | Br |
| 3.15 | $C_3H_7$-iso | H | Br |
| 3.16 | $C_4H_9$-sek | H | Br |
| 3.17 | $CH_3$ | H | Cl |
| 3.18 | $C_2H_5$ | H | Cl |
| 3.19 | $C_3H_7$-iso | H | Cl |
| 3.20 | $C_4H_9$-sek | H | Cl |
| 3.21 | $CH_3$ | H | F |
| 3.22 | $C_2H_5$ | H | F |
| 3.23 | $C_3H_7$-iso | H | F |
| 3.24 | $C_4H_9$-sek | H | F |

Tabelle 4:

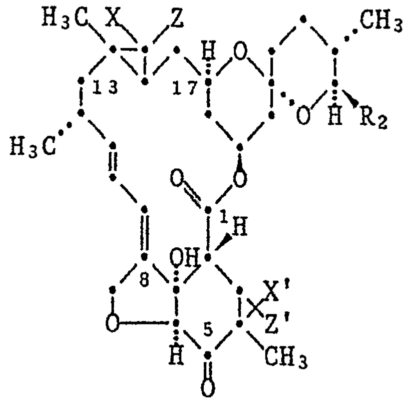

| Verb. Nr. | $R_2$ | X | Z | X' | Z' |
|---|---|---|---|---|---|
| 4.1 | $C_3H_7$-iso | Cl | Cl | Br | Br |
| 4.2 | $CH_3$ | Br | Br | Cl | Cl |
| 4.3 | $C_2H_5$ | H | Br | H | Br |
| 4.4 | $CH_3$ | H | H | H | H |
| 4.5 | $C_4H_9$-sek | Br | Br | H | H |
| 4.6 | $CH_3$ | H | Br | H | Cl |
| 4.7 | $C_3H_7$-iso | H | H | H | H |
| 4.8 | $C_2H_5$ | Cl | Cl | Cl | Cl |
| 4.9 | $C_4H_9$-sek | Cl | Cl | H | H |
| 4.10 | $C_2H_5$ | H | H | Cl | Cl |
| 4.11 | $C_2H_5$ | Br | Br | Br | Br |
| 4.12 | $CH_3$ | Cl | Cl | Cl | Cl |

Tabelle 5:

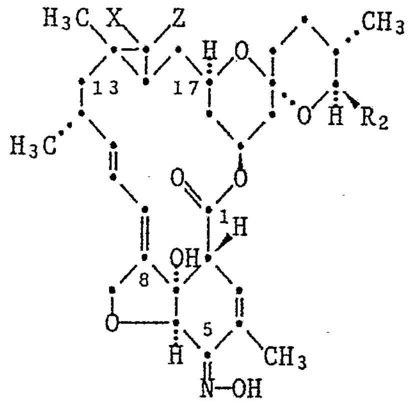

| Verb. Nr. | $R_2$ | X | Z |
|---|---|---|---|
| 5.1 | $CH_3$ | H | H |
| 5.2 | $C_2H_5$ | H | H |
| 5.3 | $C_3H_7$-iso | H | H |
| 5.4 | $C_4H_9$-sek | H | H |
| 5.5 | $CH_3$ | Br | Br |
| 5.6 | $C_2H_5$ | Br | Br |
| 5.7 | $C_3H_7$-iso | Br | Br |
| 5.8 | $C_4H_9$-sek | Br | Br |
| 5.9 | $CH_3$ | Cl | Cl |
| 5.10 | $C_2H_5$ | Cl | Cl |
| 5.11 | $C_3H_7$-iso | Cl | Cl |
| 5.12 | $C_4H_9$-sek | Cl | Cl |
| 5.13 | $CH_3$ | H | Br |
| 5.14 | $C_2H_5$ | H | Br |
| 5.15 | $C_3H_7$-iso | H | Br |
| 5.16 | $C_4H_9$-sek | H | Br |
| 5.17 | $CH_3$ | H | Cl |
| 5.18 | $C_2H_5$ | H | Cl |
| 5.19 | $C_3H_7$-iso | H | Cl |
| 5.20 | $C_4H_9$-sek | H | Cl |
| 5.21 | $CH_3$ | H | F |
| 5.22 | $C_2H_5$ | H | F |
| 5.23 | $C_3H_7$-iso | H | F |
| 5.24 | $C_4H_9$-sek | H | F |

sowie die Verbindungen der Tabelle 5, in denen das Wasserstoffatom der in 5-Position befindlichen Oximgruppe durch die Silylgruppe $-Si(CH_3)_2$-tert.-$C_4H_9$ ersetzt ist (= Verbindungen S-5.1 bis S-5.24) und die Verbindungen der Tabelle 5, in denen das Wasserstoffatom der in 5-Position befindlichen Oximgruppe durch die Acylgruppe $-COCH_3$ ersetzt ist (= Verbindungen A-5.1 bis A-5.24).

Tabelle 6:

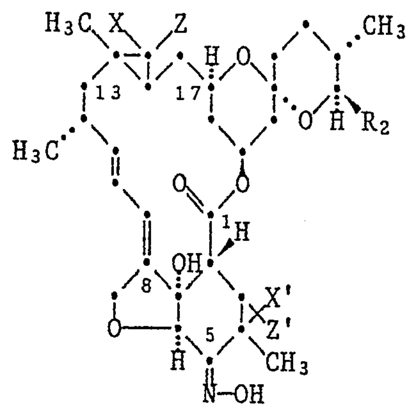

| Verb. Nr. | $R_2$ | X | Z | X' | Z' |
|---|---|---|---|---|---|
| 6.1 | $CH_3$ | Cl | Cl | Cl | Cl |
| 6.2 | $C_2H_5$ | Br | Br | Br | Br |
| 6.3 | $C_2H_5$ | H | H | H | H |
| 6.4 | $C_4H_9$-sek | Cl | Cl | H | H |
| 6.5 | $C_2H_5$ | Cl | Cl | Cl | Cl |
| 6.6 | $C_3H_7$-iso | H | H | Br | Br |
| 6.7 | $CH_3$ | H | Br | H | Br |
| 6.8 | $C_4H_9$-sek | Br | Br | Cl | Cl |
| 6.9 | $CH_3$ | H | H | H | Cl |
| 6.10 | $C_2H_5$ | H | Br | H | Br |
| 6.11 | $CH_3$ | Br | Br | H | H |
| 6.12 | $C_3H_7$-iso | Cl | Cl | Br | Br |
| 6.13 | $C_2H_5$ | Cl | Br | Br | Cl |
| 6.14 | $C_4H_9$-sek | Br | Cl | Br | Cl |

sowie die Verbindungen der Tabelle 6, in denen das Wasserstoffatom der in 5-Position befindlichen Oximgruppe durch die Silylgruppe $-Si(CH_3)_2$-tert.-$C_4H_9$ ersetzt ist (= Verbindungen S-6.1 bis S-6.14) und die Verbindungen der Tabelle 6, in denen das Wasserstoffatom der in 5-Position befindlichen Oximgruppe durch die Acylgruppe $-COCH_3$ ersetzt ist (= Verbindungen A-6.1 bis A-6.14).

Formulierungsbeispiele für den Wirkstoff der Formel I

(% = Gewichtsprozent)

| Spritzpulver | a) | b) | c) |
|---|---|---|---|
| Wirkstoff aus den Tabellen 1 bis 6 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man

erhält Spritzpulver, die sich mit Wasser zu Suspensionen jeder gewünschten Konzentration verdünnen lassen.

Emulsions-Konzentrat Wirkstoff aus den Tabellen 1 bis 6 10 %
Octylphenolpolyethylenglykolether (4-5 Mol AeO) 3 %
Ca-Dodecylbenzolsulfonat 3 %
Ricinusölpolyglykolether (36 Mol AeO) 4 %
Cyclohexanon 30 %
Xylolgemisch 50 %
Aus diesem Konzentrat können durch Verdünnen mit Wasser Emulsionen jeder gewünschten Konzentration hergestellt werden.

| Stäubemittel | a) | b) |
|---|---|---|
| Wirkstoff aus den Tabellen 1 bis 6 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Extruder Granulat Wirkstoff aus den Tabellen 1 bis 6 10 %
Na-Ligninsulfonat 2 %
Carboxymethylcellulose 1 %
Kaolin 87 %
Der Wirkstoff wird mit den Zusatzstoffen vermischt, vermahlen und mit Wasser angefeuchtet. Dieses Gemisch wird extrudiert und anschliessend im Luftstrom getrocknet.

Tabletten bzw. Boli I Ein Wirkstoff aus den Tabellen 1 bis 6 33,0 %
Methylcellulose 0,80 %
Kieselsäure hochdispers 0,80 %
Maisstärke 8,40 %
Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.
II Milchzucker krist. 22,50 %
Maisstärke 17,00 %
mikrokrist. Cellulose 16,50 %
Magnesiumstearat 1,00 %
Alle 4 Hilfsstoffe gut mischen
Phasen I und II mischen und zu Tabletten oder Boli verpressen.

Injektabiles

A. Oeliges Vehikel (langsame Freisetzung) Ein Wirkstoff aus den Tabellen 0,1-1,0 g
Erdnussöl ad 100 ml

Ein Wirkstoff aus den Tabellen 0,1-1,0 g
Sesamöl ad 100 ml
Herstellung: Der Wirkstoff wird in einem Teil des Oels unter Rühren und gegebenenfalls leichtem Erwärmen gelöst, nach Abkühlung auf das Sollvolumen aufgefüllt und durch ein geeignetes Membranfilter mit 0,22 µm sterilfiltriert.

B. Wassermischbares Lösungsmittel (mittlere Freisetzungsgeschwindigkeit) Ein Wirkstoff aus den Tabellen 0,1-1,0 g
4-Hydroxymethyl-1,3-dioxolan (Glycerol Formal) 40 g

1,2-Propandiol ad 100 ml

Ein Wirkstoff aus den Tabellen 0,1-1,0 g
Glycerindimethylketal 40 g
1,2-Propandiol ad 100 ml

Herstellung: Der Wirkstoff wird in einem Teil des Lösungsmittels unter Rühren gelöst, auf das Sollvolumen aufgefüllt und durch ein geeignetes Membranfilter mit 0.22 μm sterilfiltriert.

C. Wässriges Solubilisat (rasche Freisetzung) Ein Wirkstoff aus den Tabellen 0,1-1,0 g
Polyäthoxyliertes Ricinusöl (40 Aethylenoxideinheiten)* 10 g
1,2-Propandiol 20 g
Benzylalkohol 1 g
Aqua ad injekt. ad 100 ml

* Im Handel erhältlich unter der Bezeichnung CREMOPHOR® EL (BASF AG);

Ein Wirkstoff aus den Tabellen 0,1-1,0 g
Polyäthoxyliertes Sorbitanmonooleat (20 Aethylenoxideinheiten)** 8 g
4-Hydroxymethyl-1,3-dioxolan (Glycerol Formal) 20 g
Benzylalkohol 1 g
Aqua ad injekt. ad 100 ml

** Im Handel erhältlich unter der Bezeichnung TWEEN® 80 (ICI);

Herstellung: Der Wirkstoff wird in den Lösungsmitteln und dem Tensid gelöst und mit Wasser auf das Sollvolumen aufgefüllt. Sterilfiltration durch geeignetes Membranfilter mit 0,22 μm Porendurchmesser.

Die wässrigen System können bevorzugterweise auch für die orale und/oder intraruminale Applikation eingesetzt werden.

Bei einer Verwendung von Verbindungen der Formel I oder entsprechender Mittel zur Bekämpfung von endoparasitären Nematoden, Cestoden und Trematoden bei Haus- und Nutztieren, wie Rindern, Schafen, Ziegen, Katzen und Hunden, können die Verbindungen oder Mittel den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Die Konzentration an Wirkstoff im Fertigfutter beträgt vorzugsweise 0,005 bis 0,1 Gew. %. Die Mittel können in Form von Lösungen, Emulsionen, Suspensionen, Pulvern, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die sie enthaltenden Mittel den Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

Biologische Beispiele

B-1. Wirkung gegen $L_1$-Larven von Lucilia sericata

1 ml einer wässrigen Suspension der zu prüfenden Aktivsubstanz wird so mit 3 ml eines speziellen Larvenzuchtmediums bei ca. 50° C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Verbindungen aus den Herstellungsbeispielen, wie z.B. die Verbindungen Nr. 1.14, 1.18, 5.5 und 7.1 (Verbindung von Beispiel H-2) erzielen mit 100 ppm eine Wirkung von 100 %.

Diese Resultate werden auch beim gleichen Test gegen $L_1$-Larven von Lucilia cuprina erreicht.

B-2. Akarizide Wirkung gegen Boophilus microplus (Biarra-Stamm)

Auf einer PVC-Platte wird waagerecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jede Zecke wird mit einer Injectionsnadel 1 μl einer Flüssigkeit injiziert, die eine 1:1-Mischung von Polyethylenglykol und Aceton darstellt und in der eine bestimmte Wirkstoffmenge von wahlweise 1, 0,1 oder 0,01 μl pro Zecke gelöst ist. Kontrolltiere erhalten eine Injektion der entsprechenden Mischung ohne Wirkstoff. Nach der Behandlung werden die Tiere unter Normalbedingungen in einem Insektarium bei ca. 28° C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Verbindungen aus den Herstellungsbeispielen erzielen eine $IR_{90}$ von 1 µg.

B-3. Versuch an mit Nematoden (Haemonchus contortus und Trichostrongylus colubriformis) infizierten Schafen

Der Wirkstoff wird als Suspension formuliert mit einer Magensonde oder durch Injektion in den Pansen eines Schafes gegeben, das mit Haemonchus contortus und Trichostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 0,5 mg oder 0,1 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen aus den Herstellungsbeispielen,, wie z.B. mit der Nr. 1.14, 1.18, 2.14 oder 5.5 bei 0,1 mg/kg behandelt werden, zeigen im Vergleich zu unbehandelten, aber infizierten Vergleichsgruppen einen um 50 bis 100 % reduzierten Nematodenbefall (Reduktion der Wurmeier im Kot).

B-4. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Verbindungen aus den Tabellen 1 bis 6 bewirken in diesem Test bei einer Konzentration von 10 ppm bereits nach einem Tag eine vollständige Abtötung sämtlicher Larven.

B-5. Milbizide Wirkung gegen Dermanyssus gallinae

2 bis 3 ml einer Testlösung (100, 10, 1 und 0,1 ppm Aktivsubstanz) werden in einen nach oben offenen Glasbehälter gegeben und ca. 200 Milben in unterschiedlichen Entwicklungsstadien in diesen Behälter eingesetzt. Der Glasbehälter wird mit einem Wattebausch verschlossen und 10 Minuten gleichmässig, bis zur vollständigen Benetzung der Milben geschüttelt. Danach wird der Behälter umgekehrt hingestellt, bis die überschüssige Testlösung von der Watte aufgesogen ist. Der Behälter wird erneut gekehrt und die behandelten Milben zur Evaluierung der Wirksamkeit der Testsubstanzen drei Tage unter Laborbedingungen beobachtet, wobei die Mortalität als Massstab für Wirksamkeit herangezogen wird.

Verbindungen aus den Herstellungsbeispielen, wie beispielsweise Nr. 1.14, 1.18 und 5.5, zeigen bei 100 ppm eine Wirkung von 100 %.

**Patentansprüche**

1. Verbindungen der Formel I

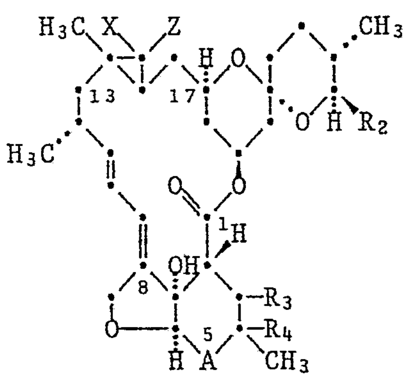

(I)

worin

A eine Gruppe

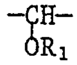 , 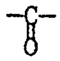 oder 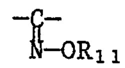

bedeutet, worin

$R_1$ für Wasserstoff oder eine OH-Schutzgruppe und $R_{11}$ für Wasserstoff, eine OH-Schutzgruppe, eine Alkyl-, Cycloalkyl- oder Acylgruppe steht,

$R_2$ Methyl, Ethyl, Isopropyl, sek.Butyl oder die Gruppe $-C(CH_3)=CH-E$ bedeutet, worin E für Methyl, Ethyl oder Isopropyl steht,

$R_3$ und $R_4$ gemeinsam eine Bindung zwischen den beiden Kohlenstoffatomen, an die sie gebunden sind, oder gemeinsam die Gruppe -C(X')(Z')-, worin X' und Z' unabhängig voneinander für Wasserstoff oder Halogen stehen, und

X und Z unabhängig voneinander Wasserstoff oder Halogen bedeuten.

2. Verbindungen der Formel I, nach Anspruch 1, worin A, $R_3$ $R_4$, X und Z die für Formel I angegebenen Bedeutungen haben und $R_2$ für Methyl, Ethyl, Isopropyl oder sek.Butyl steht.

3. Verbindungen der Formel I nach Anspruch 2, worin A eine der Gruppen $-CH(OR_1)-$, -C(O)- oder -C(=N-OH), worin $R_1$ für Wasserstoff, eine Silylgruppe oder eine Monosaccharidgruppe steht,

$R_2$ Methyl oder Ethyl, $R_3$ und $R_4$ gemeinsam eine Bindung zwischen den beiden Kohlenstoffatomen, an die sie gebunden sind, oder gemeinsam die Gruppe -C(X')(Z')-, worin X' und Z' unabhängig voneinander für Wasserstoff oder Halogen stehen, und X und Z unabhängig voneinander Wasserstoff oder Halogen bedeuten.

4. Verbindungen der Formel I nach Anspruch 2, worin A eine der Gruppen $-CH(OR_1)-$, -C(O)- oder -C(=N-OH)-, worin $R_1$ für Wasserstoff, Acetyl, tert.-Butyldimethylsilyl oder 2,3,4,6-Tetraacetylglukopyranosyl steht, $R_2$ Methyl oder Ethyl, $R_3$ und $R_4$ gemeinsam eine Bindung zwischen den beiden Kohlenstoffatomen, an die sie gebunden sind, oder gemeinsam die Gruppe $-C(Cl_2)-$, X Wasserstoff, Chlor, Brom oder Fluor und Z Wasserstoff, Chlor, Brom oder Fluor bedeuten.

5. Verbindungen der Formel I nach Anspruch 2, worin A eine der Gruppen $-CH(OR_1)-$, -C(O)- oder -C(=N-OH)-, worin $R_1$ für Wasserstoff, tert.-Butyldimethylsilyl oder 2,3,4,6-Tetraacetylglukopyranosyl steht, $R_2$ Methyl oder Ethyl, $R_3$ und $R_4$ gemeinsam eine Bindung zwischen den beiden Kohlenstoffatomen, an die sie gebunden sind, oder gemeinsam die Gruppe $-C(Cl_2)-$, X Wasserstoff, Chlor oder Brom und Z Wasserstoff, Chlor oder Brom bedeuten.

6. Verbindungen der Formel I nach Anspruch 2, worin A die Gruppe -CH(OH)- oder -C(=N-OH)-, $R_2$ Methyl oder Ethyl, $R_3$ und $R_4$ gemeinsam eine Bindung zwischen den beiden Kohlenstoffatomen, an die sie gebunden sind, X Wasserstoff, Chlor, Brom oder Fluor und Z Wasserstoff, Chlor, Brom oder Fluor bedeuten.

7. Verbindungen der Formel I nach Anspruch 2, worin A die Gruppe -CH(OH)- oder -C(=N-OH)-, $R_2$ Methyl oder Ethyl, $R_3$ und $R_4$ gemeinsam eine Bindung zwischen den beiden Kohlenstoffatomen, an die sie gebunden sind, und X und Z miteinander identisch sind und Wasserstoff, Chlor oder Brom oder X Wasserstoff und Z Chlor oder Brom bedeuten.

8. Verbindungen der Formel I nach Anspruch 2, worin A die Gruppe -CH(OH)-, $R_2$ Methyl oder Ethyl, $R_3$ und $R_4$ gemeinsam eine Bindung zwischen den beiden Kohlenstoffatomen, an die sie gebunden sind, oder gemeinsam die Gruppe $-CCl_2-$, X Wasserstoff, Chlor oder Brom und Z Wasserstoff, Chlor oder Brom bedeuten.

9. Eine Verbindung der Formel I nach Anspruch 2, ausgewählt aus der Gruppe:
5-O-(tert.-Butyldimethylsilyl)-14,15-dichlormethylen-14,15-dihydromilbemycin $A_4$,
5-O-(tert.-Butyldimethylsilyl)-3,4-dichlormethylen-14,15-dichlormethylen-3,4,14,15-tetrahydromilbemycin $A_4$,
3,4-Dichlormethylen-14,15-dichlormethylen-3,4,14,15-tetrahydromilbemycin $A_4$,
5-O-(tert.-Butyldimethylsilyl)-14,15-dibrommethylen-14,15-dihydromilbemycin $A_4$,
5-O-(2,3,4,6-Tetraacetylglukopyranosyl)-14,15-dichlormethylen-14,15-dihydromilbemycin $A_4$,
5-Oxo-14,15-dichlormethylen-14,15-dihydromilbemycin $A_4$,
5-Hydroxyimino-14,15-dichlormethylen-14,15-dihydromilbemycin $A_4$,
14,15-Dichlormethylen-14,15-dihydromilbemycin $A_3$,
5-O-(tert.-Butyldimethylsilyl)-14,15-monobrommethylen-14,15-dihydromilbemycin $A_4$,
14,15-Monobrommethylen-14,15-dihydromilbemycin $A_4$,
14,15-Monochlormethylen-14,15-dihydromilbemycin $A_4$,
5-O-(tert.-Butyldimethylsilyl)-14,15-methylen-14,15-dihydromilbemycin $A_4$,
14,15-Methylen-14,15-dihydromilbemycin $A_4$,
14,15-Dibrommethylen-14,15-dihydromilbemycin $A_4$ und
14,15-Dichlormethylen-14,15-dihydromilbemycin $A_4$.

10. Verfahren zur Herstellung von Verbindungen der Formel I

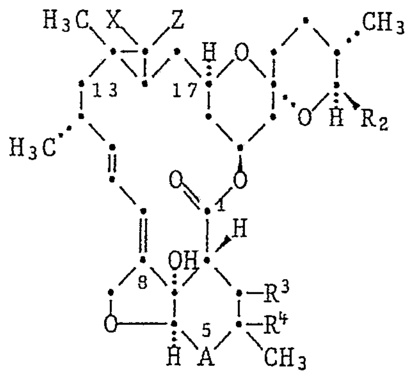

(I)

worin
A eine Gruppe

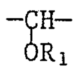 , 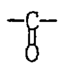 oder 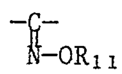

bedeutet, worin
$R_1$ für Wasserstoff oder eine OH-Schutzgruppe und $R_{11}$ für Wasserstoff, eine OH-Schutzgruppe, eine Alkyl-, Cycloalkyl- oder Acylgruppe steht,
$R_2$ Methyl, Ethyl, Isopropyl, sek.Butyl oder die Gruppe -C($CH_3$) = CH-E bedeutet, worin E für Methyl, Ethyl oder Isopropyl steht,
$R_3$ und $R_4$ gemeinsam eine Bindung zwischen den beiden Kohlenstoffatomen, an die sie gebunden sind, oder gemeinsam die Gruppe -C(X')(Z')-, worin X' und Z' unabhängig voneinander für Wasserstoff oder Halogen stehen, und
X und Z unabhängig voneinander Wasserstoff oder Halogen bedeuten, dadurch gekennzeichnet, dass man eine Verbindung der Formel II

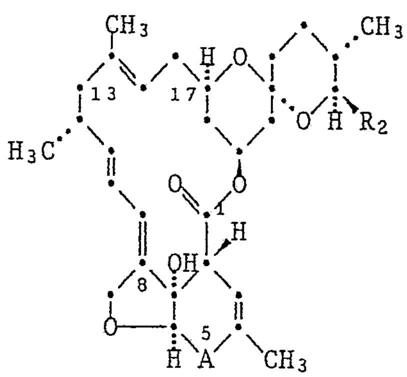

(II)

in welcher A und $R_2$ die für die Formel I angegebenen Bedeutungen haben, mit einem in situ gebildeten, in einem reaktionsinerten Lösungsmittel gelösten Carben der Formel IIIa

C(X)(Z) (IIIa)

worin X und Z die für Formel I angegebenen Bedeutungen haben, umsetzt und in erhaltenen Verbindungen gewünschtenfalls i) eine für -C(X)(Z)- stehende Gruppe -C(Halogen)(Halogen)- zur Gruppe -CH(Halogen)- oder -$CH_2$- reduziert oder ii) eine für -C(X)(Z)-stehende Gruppe -CH(Halogen)- zur Gruppe -$CH_2$- reduziert oder iii) die für A stehende Gruppe in eine andere der unter A definierten Gruppen überführt, oder erhaltene Verbindungen, in denen $R_3$ und $R_4$ gemeinsam eine Bindung zwischen den beiden Kohlenstoffatomen, an die sie gebunden sind, darstellen, mit einem in situ gebildeten, in

einem reaktionsinerten Lösungsmittel gelösten Carben der Formel IIIb
C(X')(Z') (IIIb)

worin X' und Z' die für Formel I angegebenen Bedeutungen haben, umsetzt und in erhaltenen Verbindungen gewünschtenfalls iv) eine für -C(X)(Z)- oder -C(X')(Z')- stehende Gruppe -C(Halogen)(Halogen)- zur Gruppe -CH(Halogen)- oder $-CH_2-$ reduziert oder v) eine für -C(X)(Z)-oder -C(X')(Z')-stehende Gruppe -CH(Halogen)- zur Gruppe $-CH_2-$reduziert oder vi) die für A stehende Gruppe in eine andere der unter A definierten Gruppen überführt.

11. Mittel zur Bekämpfung von Ekto- und Endoparasiten am Nutztier oder zur Bekämpfung von Schadinsekten, dadurch gekennzeichnet, dass es neben Trägermitteln, Verteilungsmitteln oder Träger- und Verteilungsmitteln als Wirkstoff mindestens eine Verbindung der Formel I gemäss Anspruch 1 enthält.

12. Mittel nach Anspruch 11, dadurch gekennzeichnet, dass es als Wirkstoff eine Verbindung der Formel I gemäss Anspruch 2 enthält.

13. Verfahren zur Bekämpfung von Nutztiere befallenden Parasiten oder von Schadinsekten, dadurch gekennzeichnet, dass man eine parasitizid oder insektizid wirksame Menge mindestens einer Verbindung der Formel I gemäss Anspruch 1 auf den Parasiten, das Schadinsekt oder deren Lebensraum appliziert.

14. Verfahren nach Anspruch 13, dadurch gekenzeichnet, dass man eine Verbindung der Formel I gemäss Anspruch 2 appliziert.